# EUROPÄISCHE PATENTANMELDUNG

(11) **EP 2 033 669 A2**
(43) Veröffentlichungstag der Anmeldung: **11.03.2009**
(21) Anmeldenummer: 08161236.8
(22) Anmeldetag: 28.07.2008
(51) Int. Cl.: A61L 31/02, A61L 31/14

(54) **Stent mit einem Grundkörper aus einer biokorrodierbaren Legierung**

(30) Priorität: 06.09.2007 DE 102007042451
(71) Anmelder: BIOTRONIK VI Patent AG, 6341 Baar (CH)
(72) Erfinder: Müller, Heinz, 91052, Erlangen (DE)
(74) Vertreter: Lindner-Vogt, Karin L.

(57) **Zusammenfassung**

Die Erfindung betrifft einen Stent mit einem Grundkörper, der ganz oder in Teilen aus einer arsen- und/oder selenhaltigen biokorrodierbaren Legierung der Elemente Magnesium, Eisen, Wolfram, Zink oder Molybdän besteht. Ein weiterer Aspekt der Erfindung liegt in der Bereitstellung eines Verfahrens zur Herstellung eines Stents mit einem Grundkörper der einen Kern und eine den Kern bedeckende Diffusionsschicht umfasst. Diese Diffusionsschicht besteht aus der genannten Legierung. Schließlich liegt ein weiterer Aspekt der Erfindung in der Bereitstellung einer arsen- und/oder selenhaltigen biokorrodierbaren Legierung der Elemente Magnesium, Eisen, Wolfram, Zink oder Molybdän.

## Beschreibung

Die Erfindung betrifft einen Stent mit einem Grundkörper, der ganz oder in Teilen aus einer biokorrodierbaren Legierung der Elemente Magnesium, Eisen, Wolfram, Zink oder Molybdän besteht. Ein weiterer Aspekt der Erfindung liegt in der Bereitstellung eines Verfahrens zur Herstellung eines solchen Stents.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper des Stents besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So können relativ kurzfristig Reizungen und Entzündungen auftreten, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich metallische Implantatwerkstoffe für Stents von Interesse, genauer biokorrodierbare Legierungen der Elemente Magnesium, Eisen, Wolfram, Zink oder Molybdän.

Eine biologische Reaktion auf metallische Elemente hängt ab von der Konzentration, Einwirkdauer und Art der Zuführung. Häufig führt allein die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn metallische Implantatwerkstoffe mit Beschichtungen aus besonders gewebeverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Derartige Beschichtungen beeinflussen jedoch auch die Degradation des Stents aus der biokorrodierbaren Legierung, so dass umfangreiche Test- und Optimierungsverfahren notwendig sind. Eine Adaption des Stents, zum Beispiel bei Änderung des Designs, des Beschichtungssystems oder der Legierungszusammensetzung erfordert einen erneuten Durchlauf der Test- und Optimierungsverfahren. Weiterhin ist die Herstellung und Handhabung derartiger beschichteter Stents aufwendig und damit mit hohen Kosten verbunden.

Trotz der erreichten Fortschritte ist eine weitere Verbesserung der Integration des Stents in sein biologisches Umfeld und dadurch Senkung der Restenoserate wünschenswert. Weiterhin besteht der Bedarf die Herstellung und Handhabung des Stents zu vereinfachen.

### Erfindungsgemäße Lösung

Ein erster Aspekt der Erfindung liegt in der Bereitstellung eines Stents mit einem Grundkörper, der ganz oder in Teilen aus einer arsen- und/oder selenhaltigen biokorrodierbaren Legierung der Elemente Magnesium, Eisen, Wolfram, Zink oder Molybdän besteht. Ein solcher Stent löst oder mindert zumindest ein oder mehrere der zuvor geschilderten Probleme.

Der Erfindung liegt unter anderem die Erkenntnis zugrunde, dass in einem gesunden Organismus ein Gleichgewicht aus Zellvermehrung (Proliferation) und Zellsterben (Apoptose) besteht. Kommt es zu einer Restenose nach Stentimplantation ist das Gleichgewicht beider Prozesse gestört und die Proliferation gewinnt überhand über den natürlichen Zelltod. Die bisherigen Strategien zur Vermeidung der Restenose setzen auf eine Hemmung der Proliferation. Histologische Präparate stenosierter Gefäße konnten jedoch keine erhöhte Konzentration an Proliferationsmarkern gegenüber dem umliegenden Gewebe belegen. Dies stützt die Annahme, dass die Apoptose weniger effektiv erfolgt als in gesundem Gewebe. Hier setzt unter anderem die Erfindung an: Das Ungleichgewicht soll durch Steigerung der Apoptoserate ausgeglichen werden. Der Vorteil gegenüber der Proliferationshemmung ist unter anderem die Vermeidung einer Akkumulierung neointimaler Zellen ohne dabei die notwendige Gewebeabdeckung des Stents zu verzögern, was häufig beim Einsatz proliferationshemmender Substanzen wie Sirolimus oder Paclitaxel beobachtet wird.

Es hat sich nun überraschenderweise gezeigt, dass der Einsatz von Selen und/oder Arsen als Bestandteil der biokorrodierbaren Legierung des Grundkörpers des Stents zur Steigerung der Apoptose führt. Der den positiven Einfluss von Arsen und/oder Selen auf die Apoptose zugrunde liegende Wirkmechanismus ist weitgehend noch ungeklärt. Vermutlich wird das am apoptotischen Vorgang beteiligte Enzym Caspase-3 aktiviert.

Der Grundkörper des Stents wird nach der Implantation allmählich abgebaut. Da die pharmakologisch aktiven Elemente in der Legierung sehr homogen verteilt sind, ist entsprechend auch das resultierende lokale Freisetzungsprofil in verschiedenen Abschnitten des Stents sehr gleichmäßig.

Sofern die Legierung nur Arsen und nicht Selen enthält, liegt vorzugsweise ein Anteil von Arsen an der Legierung im System Mg-As bei 0,01 - 40 Gew.%, insbesondere bei 0,01 - 20 Gew.%, und in den Systemen Fe-As, Mo-As, W-As und Zn-As bei 0,01 - 20 Gew.%, insbesondere 0,01 - 10 Gew.%. Besonders bevorzugt liegt der Anteil von Arsen in den genannten Legierungen bei mehr als 0,5 Gew.%, insbesondere mehr als 1 Gew.%.

Sofern die Legierung nur Selen und nicht Arsen enthält, liegt vorzugsweise ein Anteil von Selen an der Legierung im System Mg-Se bei 0,01 - 60 Gew.%, insbesondere bei 0,01 - 30 Gew.%, und in den Systemen Fe-Se, Mo-Se, W-Se und Zn-Se bei 0,01 - 30 Gew.%, insbesondere 0,01 - 15 Gew.%. Besonders bevorzugt liegt der Anteil von Selen in den genannten Legierungen bei mehr als 0,5 Gew.%, insbesondere mehr als 1 Gew.%.

Bevorzugt ist weiterhin, wenn die Anteile von Arsen und Selen an der Legierung im System Mg-As-Se bei 0,01 - 30 Gew.% As und 0,01 - 40 Gew.% Se, vorzugsweise 0, 01 - 15 Gew.% As und 0, 01 - 30 Gew.% Se liegen. Besonders bevorzugt liegt der Anteil von Arsen als auch Selen in den genannten Legierungen jeweils bei mehr als 0,5 Gew.%, insbesondere mehr als 1 Gew.%.

Bevorzugt ist ferner, wenn die Anteile von Arsen und Selen an der Legierung in den Systemen Fe-As-Se, Mo-As-Se, W-As-Se und Zn-As-Se bei 0,01 - 10 Gew.% As und 0,01 - 15 Gew.% Se, vorzugsweise 0,01 - 5 Gew.% As und 0, 01 - 10 Gew.% Se liegen. Besonders bevorzugt liegt der Anteil von Arsen als auch Selen in den genannten Legierungen jeweils bei mehr als 0,5 Gew.%, insbesondere mehr als 1 Gew.%.

Ein Gewichtsverhältnis von As zu Se in den vorgenannten arsen- und selenhaltigen Legierungen liegt vorzugsweise im Bereich von 1 : 100 bis 100 : 1, vorzugsweise 1 : 100 bis 10 : 1. Es hat sich gezeigt, dass eine Kombination von Selen und Arsen die Toxizität senkt, so dass unerwünschte Nebenreaktionen gemindert werden.

Sowohl As als auch Se bilden mit Mg, Fe, Mo, W und Zn intermetallische Phasen. Diese Phasen sind in der Regel sehr spröde und schränken sowohl die Verarbeitung als auch besonders alle Anwendungen, bei der das Implantat verformt werden muss (z.B. Stent), erheblich ein. Die Obergrenzen sind so gewählt, dass maximal die Hälfte der metallischen Matrix aus Sprödphasen besteht, so dass die für die Zwecke der vorliegenden Anwendung noch notwendige Verformbarkeit bestehen bleibt.

Der metallische Grundkörper des Stents besteht wahlweise aus einer biokorrodierbaren Magnesiumlegierung, einer biokorrodierbaren Eisenlegierung, einer biokorrodierbaren Wolframlegierung, einer biokorrodierbaren Zinklegierung oder einer biokorrodierbaren Molybdänlegierung. Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Die Legierung ist demnach so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Vorzugsweise besteht der Grundkörper aus einer biokorrodierbaren Magnesiumlegierung. Besonders bevorzugt enthält die biokorrodierbare Magnesiumlegierung neben Arsen und/oder Selen Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Besonders bevorzugt sind Magnesiumlegierungen der Zusammensetzung Seltenerdmetalle 5,2 - 9,9 Gew.%, davon Yttrium 3,7 - 5,5 Gew.%, bei denen Arsen und/oder Selen die weiter oben genannten bevorzugten Gewichtsanteile aufweisen, wobei Magnesium den auf 100 Gew.% fehlenden Anteil an der Legierung einnimmt.

Nach einer weiteren bevorzugten Ausführungsvariante, die sich insbesondere auch in Kombination mit den vorangehenden bevorzugten Ausführungsformen realisieren lässt, umfasst der Grundkörper des Stents
(a) einen Kern aus einer nicht arsen- oder selenhaltigen biokorrodierbaren Legierung der Elemente Magnesium, Eisen, Wolfram, Zink oder Molybdän; und
(b) eine den Kern bedeckende Diffusionsschicht aus der arsen- und/oder selenhaltigen biokorrodierbaren Legierung.

Eine solche Diffusionsschicht hat ein sehr hohes Haftvermögen auf dem Kern, so dass im Zuge der mechanischen Verformung des Grundkörpers des Stents beim bestimmungsgemäßen Gebrauch nicht mit einer Beschädigung der Diffusionsschicht zu rechnen ist. Die Diffusionsschicht wird mit einem geeigneten Bearbeitungsverfahren durch Abscheiden von Arsen oder Selen auf dem Kern und gleichzeitiges oder nachfolgendes Umsetzen eines oberflächennahen Teils des Kerns aus dem metallischen biokorrodierbaren Implantatwerkstoff mit zumindest Teilen dieser Abscheidung erzeugt. Mit anderen Worten, das aufgetragene Arsen oder Selen bildet mit einem oberflächennahen Teil des metallischen Implantatwerkstoffs zusammen die Diffusionsschicht aus. Die Diffusionsschicht bildet sich im Zuge des Herstellungsverfahrens durch Diffusionsvorgänge an der Phasengrenze zwischen dem metallischen Material und der arsen- oder selenhaltigen Abscheidung aus. Das sich bildende Legierungssystem der Diffusionsschicht hängt von vielen Faktoren ab, insbesondere der Temperatur und Behandlungsdauer beim Herstellungsverfahren, der Zusammensetzung des Kerns und der Zusammensetzung des zur Erstellung der Diffusionsschicht verwendeten, arsen- oder selenhaltigen Materials.

Vorzugsweise nimmt eine Konzentration von Arsen oder Selen in der Diffusionsschicht von einer Außenseite des Stents hin zum Kern ab, so dass sich die gewünschte pharmakologische Wirkung der beiden Elemente zeitnah nach der Implantation einstellt, nach Einsetzten des Heilungsvorgangs jedoch ein weitere Forcierung der Apoptose vermieden wird.

Die Diffusionsschicht weist vorzugsweise eine Schichtdicke im Bereich von 20 nm bis 50 µm, insbesondere 20 nm bis 10 µm auf.

Der Kern besteht vorzugsweise aus einer biokorrodierbaren Magnesiumlegierung. Unter Magnesiumlegierung wird hier ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung des Kerns Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Diese Magnesiumlegierung bestätigte bereits in klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Bevorzugt sind weiterhin Magnesiumlegierungen die bis zu 6 Gew.% Zink enthalten. Besonders bevorzugt ist weiterhin eine Magnesiumlegierung der Zusammensetzung Yttrium 0,5 - 10 Gew.%, Zink 0,5 - 6 Gew.%, Calcium 0,05 - 1 Gew.%, Mangan 0 - 0,5 Gew.%, Silber 0 - 1 Gew.%, Cer 0 - 1 Gew.% sowie Zirkonium 0 - 1 Gew.% oder Silizium 0 - 0,4 Gew.%, wobei sich die Angaben auf Gew.% an der Legierung beziehen und Magnesium sowie herstellungsbedingte Verunreinigungen den auf 100 Gew.% verbleibenden Restanteil an der Legierung einnehmen.

Ein weiterer Aspekt der Erfindung liegt in der Bereitstellung eines Verfahrens zur Herstellung eines Stents mit einem Grundkörper, der
(a) einen Kern aus einer nicht arsen- oder selenhaltigen biokorrodierbaren Legierung der Elemente Magnesium, Eisen, Wolfram, Zink oder Molybdän; und
(b) eine den Kern bedeckende Diffusionsschicht aus der arsen- und/oder selenhaltigen biokorrodierbaren Legierung umfasst. Das Verfahren beinhaltet die Schritte:
   (i) Bereitstellen eines Grundkörpers des Stent aus einer nicht arsen- oder selenhaltigen biokorrodierbaren Legierung der Elemente Magnesium, Eisen, Wolfram, Zink oder Molybdän;
   (ii) Kontaktieren der Oberfläche des Grundkörpers mit Selen oder Arsen in gebundener oder elementare Form; und
   (iii) gleichzeitig oder im Anschluss an Schritt (ii), thermische Behandlung des Stents zumindest im Bereich der Kontaktfläche unter Ausbildung einer arsen- oder selenhaltigen Diffusionsschicht.

Das erfindungsgemäße Verfahren sieht also vor, einen Stent aus einem biokorrodierbaren metallischen Implantatwerkstoff zu veredeln, indem eine Diffusionsschicht aus Teilen dieses Implantatwerkstoffs und Selen beziehungsweise Arsen erzeugt wird.

Das Kontaktieren im Schritt (ii) kann über ein CVD- oder ein PVD-Verfahren, Flammspritzverfahren oder Elektrolyseverfahren erfolgen. Vorzugsweise erfolgt das Kontaktieren jedoch durch Eintauchen in eine arsen- oder selenhaltige Lösung oder Dispersion.

Schritt (iii) wird vorzugsweise so durchgeführt, dass eine Konzentration von Arsen oder Selen in der entstehenden Diffusionsschicht von einer Außenseite des Stents hin zum Kern abnimmt. Diese Diffusionsschicht weist dann einen Konzentrationsgradienten für Arsen beziehungsweise Selen auf, das heißt der Atomanteil/Gewichtsanteil der Elemente in der die Diffusionsschicht bildenden Legierung nimmt hin zum Kern ab. Eine Schichtdicke der gebildeten Diffusionsschicht hängt zum Einen von der Menge des aufgetragenen Arsens oder Selens ab und zum Anderen vom Umfang der Umsetzung des aufgetragenen Materials mit dem metallischen Implantatwerkstoff des Stents im oberflächennahen Bereich der Implantatoberfläche.

Ein arsen- oder selenhaltige biokorrodierbare Legierung der Elemente Magnesium, Eisen, Wolfram, Zink oder Molybdän lässt sich mit herkömmlichen metallurgischen Verfahren herstellen. So kann beispielsweise das Zulegieren dieser Elemente schmelzmetallurgisch erfolgen, aber auch durch die Herstellung geeigneter Pulvergemische, die danach zum Halbzeug extrudiert, gesintert oder auf andere Weise in Form gebracht werden. Bei allen genannten Herstellungsverfahren, also auch dem bereits beschriebenen Diffusionsverfahren zur Herstellung einer Diffusionsschicht, liegt Arsen beziehungsweise Selen in Form von interstitiell im Gitter des Basismetalls oder der Basislegierung gelöst in Atomen oder in Form von feinen, intermetallischen Ausscheidungen vor. Hierdurch wird die bereits erwähnte homogene Verteilung beziehungsweise der eingestellte Konzentrationsgradient im Volumen des Stents erreicht.

Derartige arsen- und/oder selenhaltige biokorrodierbare Legierungen der Elemente Magnesium, Eisen, Wolfram, Zink oder Molybdän sind nach Kenntnisstand der Anmelderin bisher nicht beschrieben worden. Ein weiterer Aspekt der Erfindung liegt daher auch in der Bereitstellung der genannten Legierungen.

Nachfolgend wird die Erfindung anhand von Ausführungsbeispielen näher erläutert.

Die Beschichtung eines Implantats, insbesondere Stents, dessen Grundkörper aus einer biodegradierbaren Eisen- oder Magnesiumlegierung besteht, mit Selen, kann wie folgt durchgeführt werden:
Der Stent wird in eine Kammer mit einem Vakuum von mindestens 133,322 x 10⁻⁶ Pa (10⁻⁶ Torr) möglichst frei, auf einer elektrisch leitfähigen Halterung hängend eingebracht. In dieser Kammer befindet sich außerdem ein Selen-Target auf einem Träger, der erhitzt werden kann. Zwischen Träger und dem Stent kann eine elektrische Spannung angelegt werden.

Zur Durchführung des Prozesses wird zunächst die Vakuumkammer mit einem Inertgas (z.B. Argon) auf ca. 133,322 x 10⁻³ Pa (ca. 10⁻³ Torr) geflutet. Danach wird eine Spannung zwischen dem Stent und Träger für das Selen-Target angelegt und das Selen-Target auf ca. 200 +/- 25°C erhitzt.

Durch die angelegte Spannung wird das Selen, das vom Target abgedampft wird, auf dem Stent abgeschieden. Bei einer Prozessdauer von ca. 1 Stunde können so - in Abhängigkeit von der angelegten Spannung und der Targettemperatur - Schichtdicken zwischen 5 und 20 µm erreicht werden.

Der Stent mit der so aufgebrachten Schicht wird anschließend in einem Glühofen unter Argonatmosphäre in Abhängigkeit von der speziellen Legierung bei 450 - 525°C (Mg-Legierungen) bzw. 500 - 750°C (Eisen-Legierungen) über eine Zeit von 1 bis 72 Stunden geglüht. Zu beachten ist, dass Selen einen Schmelzpunkt von ca. 220°C besitzt. Andererseits führen aber bereits sehr geringe Gehalte der Fremdelemente, speziell hier Eisen bzw. Magnesium zu einem sehr starken Anstieg der jeweiligen Solidustemperaturen. Wird also eine geringe Menge Eisen oder Magnesium durch Interdiffusion gelöst oder wird durch ein sehr kurzes Halten knapp oberhalb ihrer Schmelztemperatur eine Eindiffusion von Magnesium- oder Eisenatomen in die schmelzflüssige Phase der Selenbeschichtung ermöglicht, kann der Diffusionsprozess zwischen einer festen Beschichtungsphase und dem Grundkörper ablaufen. Durch eine solche Glühbehandlung können Diffusionsschichten bis zu ca. 50 µm Dicke erreicht werden. Die Schichten sind in der Regel als Gradientenschichten ausgebildet. Bei kürzeren Glühzeiten verbleibt ein Teil der Beschichtung als reines Selen - abgesehen von herstellungsbedingten Verunreinigungen - auf der Oberfläche. Dies kann erwünscht sein, wenn direkt nach der Implantation hohe Dosen an Selen freigesetzt werden sollen. Bei sehr langen Glühzeiten diffundiert das Selen vollständig ein. Ein Teil des aufgebrachten Selens geht dabei immer dem System durch Sublimation verloren.

Wird bei dem oben beschriebenen Beschichtungsprozess (Sputterprozess) ein Target aus Arsen oder einer Arsen-Selen-Legierung verwendet, wird in gleicher Weise eine Arsen- oder Arsen-Selen-Diffusionsschicht erhalten. Solche Arsen-/Arsen-Selen-Targets mit Arsengehalten zwischen 0,01 und 25 Gew.% werden bereits kommerziell bei der Herstellung von halbleitenden Schichten eingesetzt. Die Prozessparameter sind im Wesentlichen übertragbar. Wegen des höheren Schmelzpunktes von Arsen (ca. 390°C) müssen die Prozessparameter bei der Diffusionsglühung entsprechend angepasst, d.h. in der Regel muss die Temperatur erhöht werden.

Zur Abscheidung der primären Arsenschicht können auch sogenannte stromlose Beschichtungsprozesse in elektrolytischen arsenhaltigen Bädern verwendet werden, bei denen Ti³⁺-lonen als Reduktionsmittel verwendet werden, in welche der Stent für eine Dauer zwischen 5 Minuten und einer Stunde getaucht wird. Solche Bäder sind technisch verfügbar. Es sind Arsen-Schichtdicken im Bereich zwischen wenigen µm bis hin zu 25 µm herstellbar. Die nachfolgende Diffusionsglühung kann wie zuvor beschrieben durchgeführt werden.

Zur Herstellung von arsen- und/oder selenhaltigen Legierungen von Eisen oder Magnesium kann ein herkömmlicher schmelzmetallurgischer Weg beschritten werden. Es ist hierbei jedoch sinnvoll, beim Erschmelzen As-Mg, As-Fe, Se-Mg bzw. Se-Fe Vorlegierungen zu verwenden um ein zu starkes Verdampfen der Komponenten Arsen bzw. Selen zu vermeiden. Die Abgusstemperaturen sollten mindestens 200°C über den Liquidustemperaturen der jeweiligen Legierungen liegen. Wegen der geringen Löslichkeit der Elemente in Mg und Fe (Ausnahme System As-Fe) sollten die Legierungen, um eine möglichst hohe Homogenität zu erreichen, möglichst schnell erstarren; z.B. in einer wassergekühlten Kokille oder durch Abguss in einen gekühlten Tiegel oder eine gekühlte Metallform.

## Patentansprüche

1. Stent mit einem Grundkörper, der ganz oder in Teilen aus einer arsen- und/oder selenhaltigen biokorrodierbaren Legierung der Elemente Magnesium (Mg), Eisen (Fe), Wolfram (W), Zink (Zn) oder Molybdän (Mo) besteht.

2. Stent nach Anspruch 1, bei dem ein Anteil von Arsen an der Legierung im System Mg-As bei 0,01 - 40 Gew.% und in den Systemen Fe-As, Mo-As, W-As und Zn-As bei 0,01 - 20 Gew.% liegt.

3. Stent nach Anspruch 1, bei dem ein Anteil von Selen an der Legierung im System Mg-Se bei 0,01 - 60 Gew.% und in den Systemen Fe-Se, Mo-Se, W-Se und Zn-Se bei 0,01 - 30 Gew.% liegt.

4. Stent nach Anspruch 1, bei dem die Anteile von Arsen und Selen an der Legierung im System Mg-As-Se bei 0,01 - 30 Gew.% As und 0,01 - 40 Gew.% Se und in den Systemen Fe-As-Se, Mo-As-Se, W-As-Se und Zn-As-Se bei 0,01 - 10 Gew.% As und 0,01 - 15 Gew.% Se liegen.

5. Stent nach Anspruch 4, bei dem ein Gewichtsverhältnis von Arsen zu Selen in der Legierung im Bereich von 1 : 100 bis 100 : 1 liegt.

6. Stent nach einem der vorhergehenden Ansprüche, bei dem der Grundkörper
(a) einen Kern aus einer nicht arsen- oder selenhaltigen biokorrodierbaren Legierung der Elemente Magnesium, Eisen, Wolfram, Zink oder Molybdän; und
(b) eine den Kern bedeckende Diffusionsschicht aus der arsen- und/oder selenhaltigen biokorrodierbaren Legierung umfasst.

7. Stent nach Anspruch 6, bei dem eine Konzentration von Arsen oder Selen in der Diffusionsschicht von einer Außenseite des Implantats hin zum Kern abnimmt.

8. Stent nach Anspruch 6 oder 7, bei dem die Diffusionsschicht eine Schichtdicke im Bereich von 20 nm bis 50 µm aufweist.

9. Verfahren zur Herstellung eines Stents mit einem Grundkörper, der
(a) einen Kern aus einer nicht arsen- oder selenhaltigen biokorrodierbaren Legierung der Elemente Magnesium, Eisen, Wolfram, Zink oder Molybdän; und
(b) eine den Kern bedeckende Diffusionsschicht aus der arsen- und/oder selenhaltigen biokorrodierbaren Legierung umfasst,
wobei das Verfahren die Schritte beinhaltet:
(i) Bereitstellen eines Grundkörpers des Stent aus einer nicht arsen- oder selenhaltigen biokorrodierbaren Legierung der Elemente Magnesium, Eisen, Wolfram, Zink oder Molybdän;
(ii) Kontaktieren der Oberfläche des Grundkörpers mit Arsen oder Selen in gebundener oder elementare Form; und
(iii) gleichzeitig oder im Anschluss an Schritt (ii), thermische Behandlung des Stents zumindest im Bereich der Kontaktfläche unter Ausbildung einer arsen- oder selenhaltigen Diffusionsschicht.

10. Verfahren nach Anspruch 9, bei dem der Schritt (iii) so durchgeführt wird, dass eine Konzentration von Arsen oder Selen in der entstehenden Diffusionsschicht von einer Außenseite des Stent hin zum Kern abnimmt.

11. Arsen- und/oder selenhaltige biokorrodierbare Legierung der Elemente Magnesium, Eisen, Wolfram, Zink oder Molybdän.
